# FASCICULE DE BREVET EUROPEEN

(11) **EP 1 367 063 B1**
(45) Date de publication et mention de la délivrance du brevet: **23.08.2006**
(21) Numéro de dépôt: 03291931.8
(22) Date de dépôt: 31.07.2003
(51) Int. Cl.: C07K 5/06, C07D 209/42

(54) **Nouveau procédé de synthèse du perindopril et de ses sels pharmaceutiquement acceptables**
Verfahren für die Synthese von Perindopril und seiner pharmazeutischen annehmbaren Salzen
Method for synthesis of perindopril and its pharmaceutically acceptable salts

(43) Date de publication de la demande: 03.12.2003
(73) Titulaire: Les Laboratoires Servier, 92415 Courbevoie Cedex (FR)
(72) Inventeur: Fugier, Claude, 76210 Gruchet le Valasse (FR); Dubuffet, Thierry, 76210 Bolbec (FR); Langlois, Pascal, 76210 Saint Jean de la Neuville (FR)

(56) Documents cités:
- WO-A-01/58868
- WO-A-03/016336
- ES-A- 8 604 144

## Description

La présente invention concerne un procédé de synthèse du perindopril de formule (I): 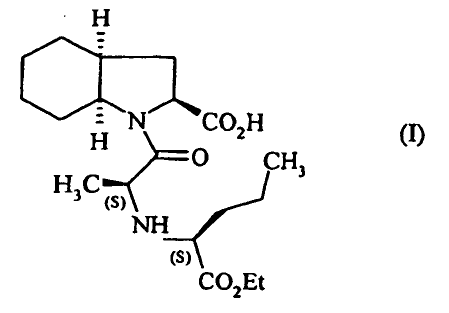 et de ses sels pharmaceutiquement acceptables.

Le perindopril, ainsi que ses sels pharmaceutiquement acceptables, et plus particulièrement son sel de tert-butylamine, possèdent des propriétés pharmacologiques intéressantes.
Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.
Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir y accéder avec un procédé de synthèse performant, facilement transposable à l'échelle industrielle, conduisant au perindopril avec un bon rendement et une excellente pureté, à partir de matières premières bon marché.
Le brevet EP 0 308 341 décrit la synthèse du perindopril par couplage de l'ester benzylique de l'acide (2*S*, 3a*S*, 7a*S*)-octahydroindole 2-carboxylique avec l'ester éthylique de la N-[(S)-1-carboxybutyl]-(S)-alanine, suivie de la déprotection du groupement carboxylique de l'hétérocycle par hydrogénation catalytique.
Or, l'ester de l'acide (2S, 3aS, 7aS)-octahydroindole-2-carboxylique n'est pas commercial, et sa préparation nécessite plusieurs étapes de synthèse (dont une étape de résolution) à partir de l'acide indole-2-carboxylique.

La demanderesse a présentement mis au point un nouveau procédé de synthèse du perindopril à partir de matières premières aisément accessibles.

Plus spécifiquement, la présente invention concerne un procédé de synthèse du perindopril, et de ses sels pharmaceutiquement acceptables, caractérisé en ce que l'on fait réagir le composé de formule (II) : 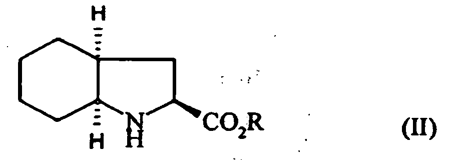 dans laquelle R représente un atome d'hydrogène ou un groupement benzyle ou alkyle (C₁-C₆) linéaire ou ramifié,
avec un composé de formule (III), de configuration (R) : 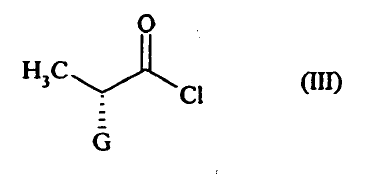 dans laquelle G représente un atome de chlore ou de brome ou un groupement hydroxy, p-toluènesulfonyloxy, méthanesulfonyloxy ou trifluorométhanesulfonyloxy,
en présence de base,
pour conduire au composé de formule (IV) : 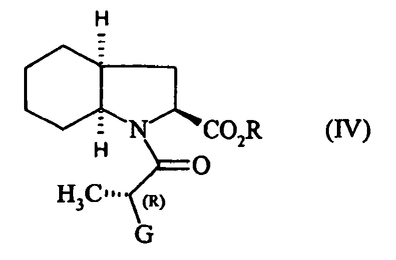 dans laquelle R et G sont tels que définis précédemment,
que l'on met en réaction avec le composé de formule (V) : 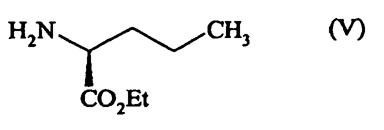 pour conduire, après déprotection le cas échéant, au composé de formule (I).

Parmi les bases utilisables pour la réaction entre les composés de formules (II) et (III), on peut citer à titre non limitatif les amines organiques telles que la triéthylamine, la pyridine ou la diisopropyléthylamine, et les bases minérales telles que NaOH, KOH, Na₂CO₃, K₂CO₃, NaHCO₃ ou KHCO₃.

Lorsque G représente un atome de chlore ou de brome, ou un groupement p-toluènesulfonyloxy, méthanesulfonyloxy ou trifluorométhanesulfonyloxy, la réaction entre les composés de formules (IV) et (V) est préférentiellement effectuée en présence d'une base, de préférence une amine organique telle que la triéthylamine, la pyridine ou la diisopropyléthylamine ou une base minérale telle que Na₂CO₃, K₂CO₃, NaHCO₃ ou KHCO₃.

Lorsque G représente un groupement hydroxy, la réaction entre les composés de formules (IV) et (V) est préférentiellement effectuée en présence d'un réactif d'activation tel que l'iodure de N-méthyl-N-phényl-aminotriphénylphosphonium, ou l'hexaméthylphosphore-triamide associé au perchlorate d'ammonium, ou, lorsque R est différent de l'atome de hydrogène, par réaction de Mitsunobu.

Les composés de formule (IV) pour lesquels G représente un atome de chlore ou un groupement p-toluènesulfonyloxy ou méthanesulfonyloxy sont des produits nouveaux, utiles comme intermédiaires de synthèse dans l'industrie chimique ou pharmaceutique, notamment dans la synthèse du perindopril, et font à ce titre partie intégrante de la présente invention.

### EXEMPLE 1 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S, 3aS, 7aS)-1-[(2R)-2-Bromopropionyl]octahydro-1H-indole-2-carboxylate de benzyle

Dans un réacteur, charger 200 g de (2S, 3aS, 7aS)-octahydro-1H-indole-2-carboxylate de benzyle, 1,5 l de dichlorométhane, puis amener la température du mélange réactionnel à 0°C et ajouter 201 ml de diisopropyléthylamine, puis 132 g de chlorure de (2R)-2-bromopropionyle. Amener ensuite le mélange à température ambiante. Après 1h d'agitation à cette température, laver le mélange à l'eau, puis avec une solution diluée d'acide acétique. La solution de (2S, 3aS, 7aS)-1-[(2R)-2-bromopropionyl]octahydro-1H-indole-2-carboxylate de benzyle ainsi obtenue est engagée telle quelle dans l'étape suivante.

### Stade B : (2S, 3aS, 7aS)-1-{(2S)-2-[(1S)-1-(Ethoxycarbonyl)-butylamino]-propionyl}-octahydro-1H-indole-2-carboxylate de benzyle

Dans un réacteur, charger 123 g de (2S)-2-aminopentanoate d'éthyle, 160 ml de triéthylamine et 160 ml d'acétonitrile, puis amener le mélange à 60°C, ajouter lentement la solution obtenue au stade A, et chauffer au reflux pendant 4h. Après retour à température ambiante, laver le mélange à l'eau et avec une solution diluée d'acide acétique, puis évaporer les solvants, pour conduire au (2S, 3aS, 7aS)-1-{(2S)-2-[(1S)-1-(éthoxycarbonyl)-butylamino]-propionyl}-octahydro-1H-indole-2-carboxylate de benzyle.

### Stade C : Acide (2S, 3aS, 7aS)-1-{(2S)-2-[(1S)-1-(éthoxycarbonyl)-butylamino]-propionyl}-octahydro-1H-indole-2-carboxylique

Dans un hydrogénateur, placer 200 g du composé obtenu dans le stade précédent, en solution dans l'acide acétique, puis 5 g de Pd/C à 10 %. Hydrogéner sous pression de 0,5 bars entre 15 et 30°C, jusqu'à absorption de la quantité théorique d'hydrogène. Eliminer le catalyseur par filtration, puis refroidir entre 0 et 5°C et récolter le solide obtenu par filtration. Laver le gâteau et le sécher jusqu'à poids constant.
L'acide (2S, 3aS, 7aS)-1-{(2S)-2-[(1S)-1-(éthoxycarbonyl)-butylamino]-propionyl}-octahydro-1*H*-indole-2-carboxylique est ainsi obtenu avec un rendement de 85 % et une pureté énantiomérique de 99 %.

### Stade D : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

Le précipité obtenu dans le stade précédent (200 g) est mis en solution dans 2,8 1 d'acétate d'éthyle, puis 40 g de tert-butylamine et 0,4 1 d'acétate d'éthyle sont ajoutés.
La suspension obtenue est ensuite portée au reflux jusqu'à dissolution totale, puis la solution obtenue est filtrée à chaud et refroidie sous agitation jusqu'à une température de 15-20°C.
Le précipité obtenu est alors filtré, réempâté à l'acétate d'éthyle, séché puis broyé pour conduire au produit attendu avec un rendement de 95%.

### EXEMPLE 2 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : Acide (2S, 3aS, 7aS)-1-[(2R)-2-bromopropionyl]octahydro-1H-indole-2-carboxylique

Dans un réacteur, charger 200 g d'acide (2S, 3aS, 7aS)-octahydro-1H-indole-2-carboxylique, 75 ml d'eau et 150 ml de toluène, puis amener le mélange entre 0 et 5°C et ajouter 250 ml de soude 5M, puis une solution de 202 g de chlorure de (2R)-2-bromopropionyle dans le toluène, tout en maintenant la température en dessous de 10°C, et le pH du milieu à 10 par ajout de soude 5M. Après 1h d'agitation supplémentaire à 10°C, ajouter de l'acide chlorhydrique concentré pour amener le pH du mélange à 6.
Séparer la phase toluénique, puis ajouter à la phase aqueuse de l'acide chlorhydrique concentré pour amener le pH à 2.
Le précipité formé est alors filtré et séché, pour conduire à l'acide (2S, 3aS, 7aS)-1-[(2R)-2-bromopropionyl]octahydro-1 H-indole-2-carboxylique.

### Stade B : Acide (2S, 3aS, 7aS)-1-{(2S)-2-[(1S)-1-(éthoxycarbonyl)-butylamino]-propionyl}-octahydro-1H-indole-2-carboxylique

Dans un réacteur, charger 105 g de (2S)-2-aminopentanoate d'éthyle, 135 ml de triéthylamine et 135 ml d'acétonitrile, puis amener le mélange à 60°C et ajouter lentement une solution de 200 g du composé obtenu au stade A dans 1,3 l de dichlorométhane, puis chauffer au reflux pendant 4h. Après retour à température ambiante, laver le mélange à l'eau et avec une solution diluée d'acide acétique, puis évaporer les solvants, pour conduire à l'acide (2S, 3aS, 7aS)-1-{(2S)-2-[(1S)-1-(éthoxycarbonyl)-butylamino]-propionyl}-octahydro-1H-indole-2-carboxylique.

### Stade C : identique au stade D de l'exemple 1.

### EXEMPLE 3 : Sel de tert-butylamine de l'acide (2S, 3aS, 7aS)-1-{(2S)-2-[(1S)-1-(éthoxycarbonyl)-butylamino]-propionyl}-octahydro-1H-indole-2-carboxylique

### Stade A : (2S, 3aS, 7aS)-1-[(2R)-2-{p-Toluènesulfonyloxy}-propionyl]-octahydro-1H-indole-2-carboxylate de benzyle

Dans un réacteur, charger 200 g de (2S, 3aS, 7aS)-octahydro-1H-indole-2-carboxylate de benzyle, 1,5 1 de dichlorométhane, puis amener la température du mélange réactionnel à 0°C et ajouter 201 ml de diisopropyléthylamine, puis 202 g de chlorure de (1R)-2-chloro-1-méthyl-2-oxoéthyl-p-toluènesulfonate. Amener ensuite le mélange à température ambiante. Après 1h d'agitation à cette température, laver le mélange à l'eau. La solution de (2S, 3aS, 7aS)-1-[(2R)-2-{p-toluènesulfonyloxy}-propionyl]-octahydro-1H-indole-2-carboxylate de benzyle ainsi obtenue est engagée telle quelle dans l'étape suivante.

*Stades B à D : identiques aux stades B à D de l'exemple 1.*

## Revendications

1. Procédé de synthèse du composé de formule (I): 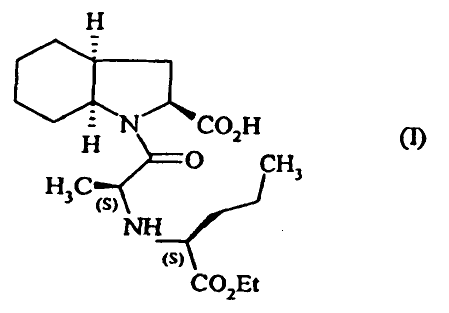 et de ses sels pharmaceutiquement acceptables,
**caractérisé en ce que** l'on fait réagir le composé de formule (II): 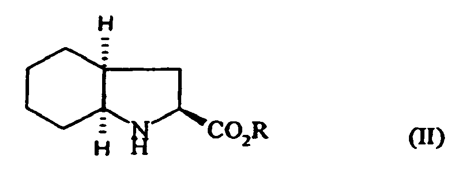 dans laquelle R représente un atome d'hydrogène ou un groupement benzyle ou alkyle (C₁-C₆) linéaire ou ramifié,
avec un composé de formule (III), de configuration (R) : 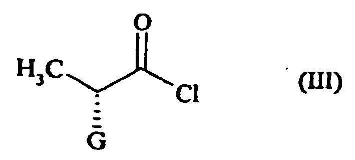 dans laquelle G représente un atome de chlore ou de brome ou un groupement hydroxy, p-toluènesulfonyloxy, méthanesulfonyloxy ou trifluorométhanesulfonyloxy,
en présence de base,
pour conduire au composé de formule (IV) : 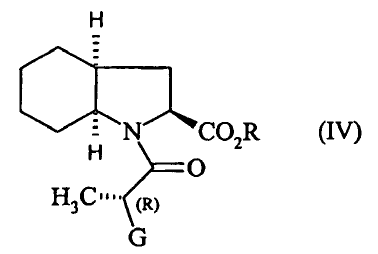 dans laquelle R et G sont tels que définis précédemment,
que l'on met en réaction avec le composé de formule (V) : 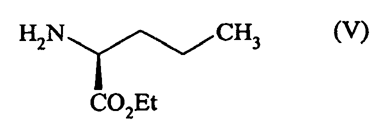 pour conduire, après déprotection le cas échéant, au composé de formule (I).

2. Procédé de synthèse selon la revendication 1, **caractérisé en ce que** la base utilisée pour la réaction entre les composés de formules (II) et (III) est une amine organique choisie parmi la triéthylamine, la pyridine et la diisopropyléthylamine, ou une base minérale choisie parmi NaOH, KOH, Na₂CO₃, K₂CO₃, NaHCO₃ et KHCO₃.

3. Procédé de synthèse selon la revendication 1, **caractérisé en ce que** G représente un atome de chlore ou de brome, ou un groupement p-toluènesulfonyloxy, méthanesulfonyloxy ou trifluorométhanesulfonyloxy.

4. Procédé de synthèse selon la revendication 3, **caractérisé en ce que** la réaction entre les composés de formules (IV) et (V) est effectuée en présence d'une amine organique choisie parmi la triéthylamine, la pyridine et la diisopropyléthylamine, ou une base minérale choisie parmi Na₂CO₃, K₂CO₃, NaHCO₃ et KHCO₃.

5. Procédé de synthèse selon la revendication 1, **caractérisé en ce que** G représente un groupement hydroxy.

6. Procédé de synthèse selon la revendication 5, **caractérisé en ce que** la réaction entre les composés de formules (IV) et (V) est effectuée en présence d'un réactif d'activation choisi parmi l'iodure de N-méthyl-N-phényl-aminotriphénylphosphonium et l'hexaméthylphosphoretriamide associé au perchlorate d'ammonium, ou, lorsque R est différent de l'atome de hydrogène, par réaction de Mitsunobu.

7. Composé de formule (IV) : 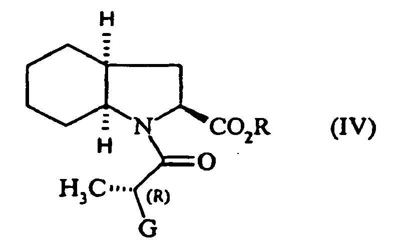 dans laquelle R représente un atome d'hydrogène ou un groupement benzyle ou alkyle (C₁-C₆) linéaire ou ramifié, et G représente un atome de chlore ou un groupement p-toluènesulfonyloxy ou méthanesulfonyloxy.

8. Procédé de synthèse selon l'une quelconque des revendications 1 à 6 du perindopril sous sa forme de sel de tert-butylamine.

## Claims

1. Process for the synthesis of the compound of formula (I) : 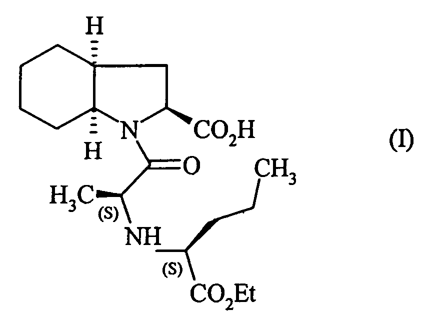 and its pharmaceutically acceptable salts,
**characterised in that** a compound of formula (II) : 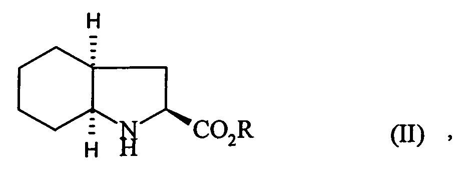 wherein R represents a hydrogen atom or a benzyl or linear or branched (C₁-C₆)alkyl group,
is reacted with a compound of formula (III) having the (R) configuration : 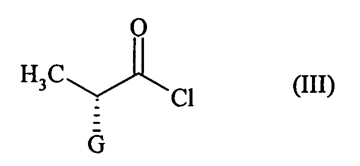 wherein G represents a chlorine or bromine atom or a hydroxy, p-toluenesulphonyloxy, methanesulphonyloxy or trifluoromethanesulphonyloxy group,
in the presence of a base,
to yield a compound of formula (IV) : 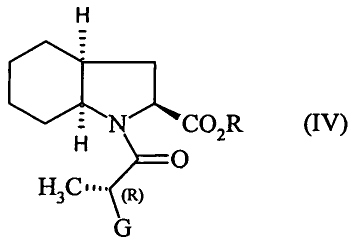 wherein R and G are as defined hereinbefore,
which is reacted with the compound of formula (V) : 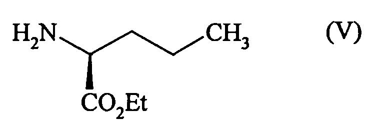 to yield, after deprotection where necessary, the compound of formula (I).

2. Synthesis process according to claim 1, **characterised in that** the base used for the reaction between the compounds of formulae (II) and (III) is an organic amine selected from triethylamine, pyridine and diisopropylethylamine, or a mineral base selected from NaOH, KOH, Na₂CO₃, K₂CO₃, NaHCO₃ and KHCO₃.

3. Synthesis process according to claim 1, **characterised in that** G represents a chlorine or bromine atom, or a p-toluenesulphonyloxy, methanesulphonyloxy or trifluoromethanesulphonyloxy group.

4. Synthesis process according to claim 3, **characterised in that** the reaction between the compounds of formulae (IV) and (V) is carried out in the presence of an organic amine selected from triethylamine, pyridine and diisopropylethylamine, or of a mineral base selected from Na₂CO₃, K₂CO₃, NaHCO₃ and KHCO₃.

5. Synthesis process according to claim 1, **characterised in that** G represents a hydroxy group.

6. Synthesis process according to claim 5, **characterised in that** the reaction between the compounds of formulae (IV) and (V) is carried out in the presence of an activation reagent selected from N-methyl-N-phenyl-aminotriphenylphosphonium iodide and hexamethylphosphorus triamide together with ammonium perchlorate, or, when R is other than a hydrogen atom, by Mitsunobu reaction.

7. Compound of formula (IV) : 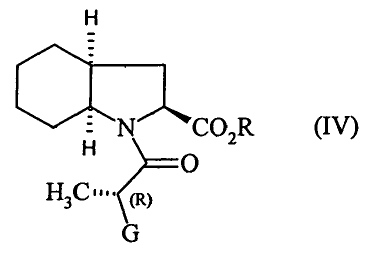 wherein R represents a hydrogen atom or a benzyl or linear or branched (C₁-C₆)alkyl group and G represents a chlorine atom or a p-toluenesulphonyloxy or methanesulphonyloxy group.

8. Process according to any one of claims 1 to 6 for the synthesis of perindopril in the form of its tert-butylamine salt.

## Patentansprüche

1. Verfahren zur Synthese von der Verbindung der Formel (I): 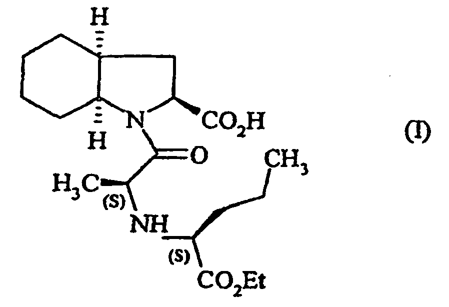 und von dessen pharmazeutisch annehmbaren Salzen,
**dadurch gekennzeichnet, daß** man die Verbindung der Formel (II): 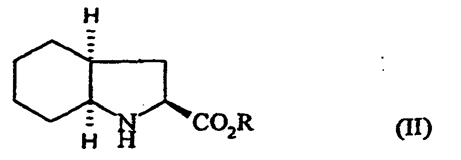 in der R ein Wasserstoffatom oder eine Benzylgruppe oder eine geradkettige oder verzweigte (C₁-C₆)-Alkylgruppe bedeutet,
mit einer Verbindung der Formel (III), der Konfiguration (R): 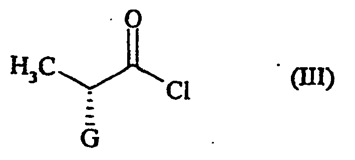 in der G ein Chloratom, ein Bromatom, eine Hydroxygruppe, p-Toluolsulfonyloxygruppe, Methansulfonyloxygruppe oder Trifluormethansulfonyloxygruppe bedeutet,
in Gegenwart einer Base umsetzt,
zur Bildung der Verbindung der Formel (IV): 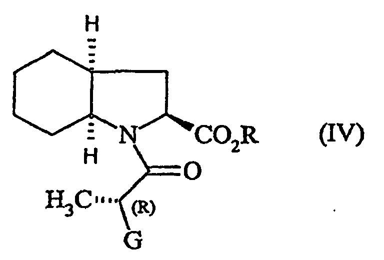 in der R und G die oben angegebenen Bedeutungen besitzen,
welche man mit der Verbindung der Formel (V): 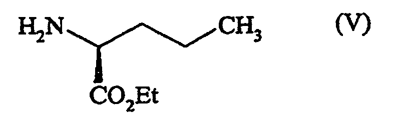 umsetzt, so daß man gegebenenfalls nach der Abspaltung der Schutzgruppen die Verbindung der Formel (I) erhält.

2. Syntheseverfahren nach Anspruch 1, **dadurch gekennzeichnet, daß** man als Base für die Umsetzung der Verbindungen der Formel (II) und (III) ein organisches Amin, ausgewählt aus Triethylamin, Pyridin und Diisopropylethylamin, oder eine anorganische Base, ausgewählt aus NaOH, KOH, Na₂CO₃, K₂CO₃, NaHCO₃ und KHCO₃, verwendet.

3. Syntheseverfahren nach Anspruch 1, **dadurch gekennzeichnet, daß** G ein Chloratom, ein Bromatom oder eine p-Toluolsulfonyloxygruppe, Methansulfonyloxygruppe oder Trifluormethansulfonyloxygruppe bedeutet.

4. Syntheseverfahren nach Anspruch 3, **dadurch gekennzeichnet, daß** die Umsetzung der Verbindungen der Formeln (IV) und (V) in Gegenwart eines organischen Amins, ausgewählt aus Triethylamin, Pyridin und Diisopropylethylamin, oder einer anorganischen Base, ausgewählt aus Na₂CO₃, K₂CO₃, NaHCO₃ und KHCO₃, durchgeführt wird.

5. Syntheseverfahren nach Anspruch 1, **dadurch gekennzeichnet, daß** G eine Hydroxygruppe bedeutet.

6. Syntheseverfahren nach Anspruch 5, **dadurch gekennzeichnet, daß** die Umsetzung der Verbindungen der Formeln (IV) und (V) in Gegenwart eines Aktivierungsreagens, ausgewählt aus N-Methyl-N-phenyl-aminotriphenylphosphoniumiodid und Hexamethylphosphortriamid in Kombination mit Ammoniumperchlorat oder, wenn R von Wasserstoff verschieden ist, durch eine Mitsunobu-Reaktion durchgeführt wird.

7. Verbindung der Formel (IV): 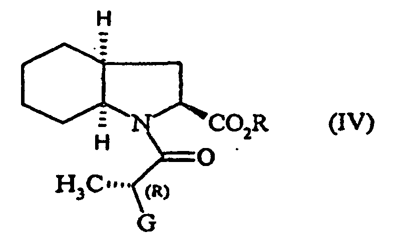 in der R ein Wasserstoffatom, eine Benzylgruppe oder eine geradkettige oder verzweigte (C₁-C₆)-Alkylgruppe und G ein Chloratom oder eine p-Toluolsulfonyloxygruppe oder Methansulfonyloxygruppe bedeuten.

8. Syntheseverfahren nach einem der Ansprüche 1 bis 6 zur Herstellung von Perindopril in Form seines tert.-Butylaminsalzes.
